# EUROPÄISCHE PATENTSCHRIFT

(11) **EP 3 795 814 B1**
(45) Veröffentlichungstag und Bekanntmachung des Hinweises auf die Patenterteilung: **02.04.2025**
(21) Anmeldenummer: 20194826.2
(22) Anmeldetag: 07.09.2020
(51) Int. Cl.: F02D 19/06, F02D 41/00, F02D 41/14

(54) **VERFAHREN ZUR SENSIERUNG EINER KRAFTSTOFFZUSAMMENSETZUNG ZUR EINSCHRÄNKUNG DER NUTZBARKEIT EINES FAHRZEUGES BEI EINER FEHLBETANKUNG**
METHOD FOR SENSING A FUEL COMPOSITION TO RESTRICT THE USABILITY OF A VEHICLE IN THE EVENT OF MISFUELING
PROCÉDÉ DE DÉTECTION D'UNE COMPOSITION DE CARBURANT PERMETTANT DE LIMITER LA POSSIBILITÉ D'UTILISATION D'UN VÉHICULE EN CAS D'UN RAVITAILLEMENT INCORRECT

(30) Priorität: 18.09.2019 DE 102019125083
(43) Veröffentlichungstag der Anmeldung: 24.03.2021
(73) Patentinhaber: VOLKSWAGEN AG, 38440 Wolfsburg (DE)
(72) Erfinder: GOBRECHT, Stefan, 38518 Gifhorn (DE)
(74) Vertreter: Gulde & Partner

(56) Entgegenhaltungen:
- WO-A1-2019/129858
- DE-A1- 102008 002 356
- DE-A1- 102008 002 476
- DE-A1- 102012 019 609
- DE-A1- 102013 215 224
- DE-B3- 102008 006 798
- JOINT RESEARCH CENTER: "The European Commission's science and knowledge service", 30 November 2018 (2018-11-30), pages 1 - 47, XP093036556, Retrieved from the Internet <URL:https://climate.ec.europa.eu/document/download/76f0d05f-21cb-46f9-af06-e1f478fdccce_en?filename=201811_engine_en.pdf> [retrieved on 20230331]

## Beschreibung

Die Erfindung betrifft ein Verfahren zur CO₂-Zertifizierung und Homologation von Fahrzeugen unter Berücksichtigung mindestens eines Gestaltungsmerkmales des Fahrzeuges.

Als Stand der Technik werden die DE 10 2008 002 476 A1, DE 10 2008 002 356 A1, DE 10 2008 006 798 B3, WO 2019/129858 A1, DE 10 2013 215 224 A1, DE 10 2008 014 613 A1, EP 1 847 704 A1, DE 10 2017 203 849 A1, DE 10 2012 019 609 A1 und DE 10 2009 028 321 A1 genannt. Zudem wird auf eine Veröffentlichung des Joint Research Center: "The European Commission's science and knowledge service", November 2018, Seiten 1-47 hingewiesen.

Der Erfindung liegt nun die Aufgabe zugrunde, ein Verfahren und ein zugehöriges System zu schaffen, welche den Betrieb des Fahrzeuges auf der Basis einer Kraftstoffzusammensetzung beeinflussen. Ziel ist es, den positiven Effekt der CO₂-Gesamtbilanz einiger Kraftstoffe in der Anwendung wirksamer zu nutzen.

Kraftstoffe enthalten immer höhere Bio- und Synthetikanteile. Einer der wesentlichen Vorteile dieser Kraftstoffe besteht in den verbesserten CO₂-Werten der Gesamtbilanz. Beispielhaft wird der Bio-Kraftstoff R33 der Anmelderin genannt. R33 BlueDiesel entspricht der Dieselnorm DIN EN 590. Der Kraftstoff besteht bis zu einem Drittel aus erneuerbaren Komponenten und hat im Vergleich zu herkömmlichem Diesel das Potenzial, mindestens 20 % an CO₂ einzusparen.

Bekannt sind auch andere CO₂-reduzierte Biokraftstoffe, wie paraffinischer Diesel und Sweden Class, der Bio-Ethanol-Anteile aufweist sowie Diesel-Kraftstoffe, dem OME (Polyoxymethylendimethylether) als Additiv beigemischt sind.

Derzeit werden die Vorteile, die neue Kraftstoffe mit verbesserter CO₂-Bilanz bringen, immer über Verteilschlüssel auf die Flottenverbräuche eines Herstellers umgerechnet.

Mit anderen Worten, die Verwendung eines CO₂-reduzierten Biokraftstoffes wird nicht dem einzelnen Fahrzeug zugeordnet. In bekannter Weise müssen neue Fahrzeuge, Fahrzeugkomponenten oder Fahrzeugsysteme vor Markteinführung entsprechend den einschlägigen Normen und Regeln des jeweiligen Bestimmungslandes homologisiert werden. Dabei findet beispielsweise die Verwendung von CO₂-reduzierten Biokraftstoffen keine Berücksichtigung.

Ausgangspunkt der Erfindung ist ein Verfahren zur CO₂-Zertifizierung und CO₂-abhängigen Homologation von Fahrzeugen unter Berücksichtigung mindestens eines Gestaltungsmerkmales des Fahrzeuges.

Wenn gemäß der Erfindung in den Ansprüchen und der Beschreibung von Fahrzeugen gesprochen wird, so werden Kraft-, Schienen-, Luft- oder Wasserfahrzeuge gleichermaßen angesprochen.

Erfindungsgemäß ist vorgesehen, dass als Gestaltungsmerkmal des Fahrzeuges die festgestellte Verwendung eines CO₂-reduzierten Kraftstoffs berücksichtigt wird,
wobei über eine Motorsteuerung Signale eines Kraftstoff-Sensors dahingehend geprüft werden, ob sich der CO₂-reduzierte Kraftstoff im Kraftstofftank befindet, für den das Fahrzeug auch homologisiert wurde,
- wobei eine festgestellte Verwendung des CO₂-reduzierten Kraftstoffs anhand eines ermittelten spezifischen Zusammensetzungs-Musters berücksichtigt wird, wobei ein Kraftstoff-Sensor in einem Kraftstofftank oder in einer Kraftstoffzuführungsleitung zwischen dem Kraftstofftank und einer Brennkraftmaschine in einem Kraftstoffversorgungssystem angeordnet ist,
- wobei der Kraftstoff-Sensor eingerichtet ist, chemische Bestandteile des Kraftstoffs zu ermitteln, um das spezifische Zusammensetzungs-Muster zu ermitteln, anhand dessen eine Unterscheidung durch Gegenüberstellung mit mindestens einem Vergleichs-Zusammensetzungs-Muster vorgenommen wird,
- wobei, wenn das spezifische Zusammensetzungs-Muster mit dem Vergleichs-Zusammensetzungs-Muster übereinstimmt, durch Vergleich der Muster nicht-CO₂-reduzierter Kraftstoff von CO₂-reduziertem Kraftstoff unterschieden wird,
- wobei die ausschließliche Verwendung des CO₂-reduzierten Kraftstoffs in dem Fahrzeug weitestgehend sichergestellt wird, indem eine Benutzung des Fahrzeuges nur unter Verwendung des CO₂-reduzierten Kraftstoffs erlaubt wird, wobei
- einem Benutzer in dem Fall, dass mittels des Kraftstoff-Sensors festgestellt wird, dass sich kein CO₂-reduzierter Kraftstoff im Kraftstofftank befindet über die Motorsteuerung Maßnahmen durchgeführt werden , wobei über mindestens ein Anzeigeelement ein Hinweis zur aktuellen Nichtverwendung des CO₂-reduzierten Kraftstoffs gestuft ausgegeben wird, wobei mindestens ein Hinweis zumindest eine Information über die erfolgte Fehlbetankung und eine Aufforderung zu einer korrigierenden Handlung umfasst, wobei
- a) in dem mindestens einen Anzeigeelement des Kraftstoffversorgungssystems des Fahrzeuges in einer ersten Stufe über eine durch den Kraftstoff-Sensor festgestellte Fehlbetankung informiert wird, wobei dazu aufgefordert wird, innerhalb einer vorgebbaren Fahrstrecke die nächste Werkstatt aufzusuchen, oder
- b) in dem mindestens einen Anzeigeelement des Kraftstoffversorgungssystems des Fahrzeuges in einer zweiten Stufe über die durch den Kraftstoff-Sensor festgestellte Fehlbetankung informiert wird, wobei dazu aufgefordert wird, innerhalb der vorgebbaren Fahrstrecke die nächste Werkstatt aufzusuchen, wobei ferner in dem Anzeigeelement des Kraftstoffversorgungssystems des Fahrzeuges über durch die Fehlbetankung zukünftig eintretende einschränkende Maßnahmen informiert wird, wobei
- hinsichtlich der Vermeidung der Umgehbarkeit der gestuften Hinweise wirksamkeitsgestufte Maßnahmen eingeleitet werden, wie
- Z1: eine Begrenzung einer Höchstgeschwindigkeit des Fahrzeuges beziehungsweise einer Drehzahl der Brennkraftmaschine und/oder
- Z2: eine Reduzierung des Moments der Brennkraftmaschine und/oder
- Z3: eine Verhinderung eines vorgebbaren n-ten Motorstarts, wobei n = 0 sein kann, so dass bereits ein nächster Motorstart verhindert wird, oder
- Z4: eine Verhinderung des vorgebbaren n-ten Motorstarts in Kombination mit dem Erreichen der vorgegebenen Fahrtstrecke, wobei nach dem Erreichen der vorgegebenen Fahrtstrecke nur n-vorgebbare Motorstarts erlaubt ist/sind, wobei n = 0 sein kann, so dass nach dem Erreichen der vorgegebenen Fahrtstrecke bereits ein nächster Motorstart verhindert wird.

Es wird weiter vorgeschlagen, dass mittels des Kraftstoff-Sensors objektiv die chemische Zusammensetzung ermittelt beziehungsweise gemessen wird. In einer bestimmten Zusammensetzung wird in einer Art spezifischen chemischen Zusammensetzungs-Muster ein Kraftstoff bestimmt, der durch Vergleich mit mindestens einem normierten spezifischen Zusammensetzungs-Muster, einem sogenannten Vergleichs-Zusammensetzungs-Muster, als CO₂-reduzierter Kraftstoff gilt oder festgelegt wird, wenn das spezifische Zusammensetzungs-Muster mit dem Vergleichs-Zusammensetzungs-Muster übereinstimmt, so dass über die spezifischen chemischen Zusammensetzungs-Muster und durch Vergleich mit mindestens einem normierten Vergleichs-Zusammensetzungs-Muster nicht-CO₂-reduzierter Kraftstoff von CO₂-reduziertem Kraftstoff unterschieden werden kann. Es wird somit mindestens ein genormter Bio-Kraftstoff, bevorzugt mehrere genormte Bio-Kraftstoffe als CO₂-reduzierte Kraftstoffe festgelegt, die man anhand der chemischen Zusammensetzung und/oder anhand von Markern erkennen/analysieren kann, wobei diese/r genormte/n Bio-Kraftstoff/e als CO₂-reduzierte/r Kraftstoff/e angesehen und bezeichnet wird/werden, und jeder der CO₂-reduzierten Kraftstoffe als Vergleichs-Zusammensetzungs-Muster zum Vergleich mit anderen Kraftstoffen zur Verfügung steht.

In bevorzugter Ausgestaltung der Erfindung ist vorgesehen, dass weitere CO₂-reduzierende Gestaltungsmerkmale der Aerodynamik und/oder des Leichtbaus und/oder des Wirkungsgrads des Motors und/oder, insbesondere die Verwendung eines ökologisch erzeugten Ökostromanteiles, insbesondere eines Plug-in-Hybrid-Fahrzeuges, des zu zertifizierenden und/oder zu homologisierenden Fahrzeuges festgestellt und bei der CO₂-Zertifizierung und CO₂-abhängigen Homologation des Fahrzeuges berücksichtigt werden.

Insbesondere wird die Unterscheidung eines nicht-CO₂-reduzierten Kraftstoffs von einem CO₂-reduzierten Kraftstoff anhand von chemischen Bestandteilen des spezifischen Zusammensetzungs-Musters vorgenommen, wobei diese chemischen Bestandteile auch im normierten Vergleichs-Zusammensetzungs-Muster entsprechend vorhanden sind.

In einer anderen Ausgestaltung des Verfahrens wird die Unterscheidung eines nicht-CO₂-reduzierten Kraftstoffs von einem CO₂-reduzierten Kraftstoff anhand mindestens eines Markers des spezifischen Zusammensetzungs-Musters vorgenommen, der einem als CO₂-reduzierten Kraftstoff zugelassenen Kraftstoff hinzugefügt wird, wobei der mindestens eine Marker auch im normierten Vergleichs-Zusammensetzungs-Muster entsprechend vorhanden ist.

Die beiden Verfahrensausgestaltungen können miteinander kombiniert werden.

Insofern wird bevorzugt ein Verfahren vorgeschlagen, bei dem die chemischen Bestandteile des Kraftstoffs oder des mindestens einen Markers durch ein Analyseverfahren festgestellt werden, welches dem mindestens einen Kraftstoff-Sensor zugeordnet ist, der die chemischen Bestandteile und/oder den mindestens einen dem zugelassenen Kraftstoff hinzugefügten Marker auf chemische oder andere Weise erkennt und das spezifische Zusammensetzungs-Muster gebildet wird, mittels dem durch den oben erläuterten Vergleich mit dem Vergleichs-Zusammensetzungs-Muster ein Rückschluss auf den CO₂-reduzierten Kraftstoff gelingt.

Es versteht sich, dass gesetzliche Regularien und Normen vorliegen und zu Rate gezogen werden, welche die chemische Kraftstoffzusammensetzung, das heißt die spezifischen Vergleichs-Zusammensetzungs-Muster von etablierten CO₂-reduzierten Kraftstoffen enthalten beziehungsweise wiederspiegeln.

Vorgesehen ist ferner bevorzugt, dass ein vorgebbarer Schwellenwert derjenigen Anteile der chemischen Bestandteile des Kraftstoffs festgelegt wird, wobei der Schwellenwert überschritten werden muss, damit der Kraftstoff mittels des spezifischen Vergleichs-Zusammensetzungs-Musters als CO₂-reduzierter Kraftstoff erkannt und eingestuft wird.

Es ist vorgesehen, dass als Analyseverfahren zur Analyse der chemischen Bestandteile des Kraftstoffs und/oder des mindestens eines Markers ein Spektroskopieverfahren, wie
- eine NIR-Spektroskopie (NIR =Nahinfrarot-Spektroskopie) oder
- eine NMR-Spektroskopie (NMR = Nuklear Magnet-Resonanz-Spektroskopie) oder
- eine LIF-Spektroskopie (LIF = Laserinduzierte Fluoreszenz-Spektroskopie) oder
- eine ZLIF-Spektroskopie (ZLIF = zeitliche laserinduzierte Fuoreszenz-Spektroskopie)
durchgeführt wird, welchem ein Kraftstoff-Sensor zugeordnet ist, der die chemischen Bestandteile des Kraftstoffs und des mindestens einen Markers feststellt.

Insbesondere bei der Durchführung der LIF-Spektroskopie oder der ZLIF-Spektroskopie ist dem Kraftstoff-Sensor innerhalb des Kraftstoffversorgungssystems optinal eine in den Figur nicht näher dargestellte Verdünnungseinheit zugeordnet, um die zu analysierende Probe zu verdünnen, wodurch bei der Auswertung der chemischen Bestandteile des Kraftstoffs eine höhere Trennschärfe erreicht wird.

In einer anderen Ausgestaltung ist vorgesehen, dass mindestens eines der zuvor genannten Analyseverfahren oder ein anderes markerspezifsiches Analyseverfahren zur Analyse des mindestens einen Markers ein dem jeweiligen Marker spezifisch zugeordnetes Analyseverfahren durchgeführt wird, welchem ein spezifischer Kraftstoff-Sensor zugeordnet ist, der ausschließlich den mindestens einen Marker auf andere nichtchemische spezifische Weise erkennt beziehungsweise feststellt.

Das Verfahren wird ferner derart augebildet, dass ausschließlich eine Verwendung eines CO₂-reduzierten Kraftstoffs in dem Fahrzeug weitestgehend sichergestellt wird, indem eine Benutzung des Fahrzeuges nur unter Verwendung des CO₂-reduzierten Kraftstoffs erlaubt wird.

Erfindungsgemäß wird eine Hinweis- und Aufforderungfunktion implementiert.

Das Verfahren ist beispielsweise dadurch gekennzeichnet, dass dem Benutzer in dem Fall, dass mittels des Kraftstoff-Sensors festgestellt wird, dass sich kein CO₂-reduzierter Kraftstoff im Kraftstofftank befindet und/oder für den Fall, dass an einer Stromladesäule kein Strom mit einem Ökostrom-Anteil verwendet wurde, über mindestens ein Anzeigeelement ein Hinweis zur aktuellen Nichtverwendung des CO₂-reduzierten Kraftstoffs und/oder zur Nichtverwendung eines Stroms mit zumindest einem bestimmten Ökostrom-Anteil ausgegeben, insbesondere gestuft ausgegeben wird, wobei ein Hinweis zumindest eine Information über die erfolgte Fehlbetankung (Kraftstoff und/oder Strom) und eine Aufforderung zu einer korrigierenden Handlung umfasst.

Mit anderen Worten, die Verwendung von Ladestrom mit einem bestimmten vorgegebenen für das Fahrzeug normierten Ökostrom-Anteils wird analog zu dem als CO₂-reduzierten Kraftstoff normierten Kraftstoff zur Homologation des Fahrzeuges verwenden.

Die nachfolgend erläuterten Maßnahmen, werden beispielsweise ergriffen, wenn kein Ladestrom mit dem bestimmten vorgegebenen normierten Ökostrom-Anteil und/oder wenn kein als normierter CO₂-reduzierter geltender Kraftstoff verwendet worden ist.

Erfindungsgemäß wird eine Maßnahme-Informationsfunktion nach Anspruch 1 implementiert.

Insbesondere ist als Informations-Maßnahme bevorzugt vorgesehen, dass a) in dem mindestens einen Anzeigeelement eines Kraftstoffversorgungssystems des Fahrzeuges in einer ersten Stufe über eine durch den Kraftstoff-Sensor festgestellte Fehlbetankung informiert wird, wobei dazu aufgefordert wird, innerhalb einer vorgebbaren Fahrstrecke die nächste Werkstatt aufzusuchen.

Oder es ist als Informations-Maßnahme bevorzugt vorgesehen, dass b) in dem mindestens einen Anzeigeelement eines Kraftstoffversorgungssystems des Fahrzeuges in einer zweiten Stufe über eine durch den Kraftstoff-Sensor festgestellte Fehlbetankung informiert wird, wobei dazu aufgefordert wird, innerhalb einer vorgebbaren Fahrstrecke die nächste Werkstatt aufzusuchen, wobei ferner in dem Anzeigeelement des Kraftstoffversorgungssystems des Fahrzeuges über durch die Fehlbetankung zukünftig eintretende einschränkende Maßnahmen informiert wird.

Erfindungsgemäß wird die Maßnahmefunktion implementiert, die aktiv in bestimmte Parameter des Fahrzeuges eingreift, ohne dabei den Nutzer des Fahrzeuges zu gefährden.

Gemäß Anspruch 1 ist vorgesehen, dass hinsichtlich der Vermeidung der Umgehbarkeit des mindestens einen der gestuften Hinweise a), b) die wirksamkeitsgestuften Maßnahmen eingeleitet werden.

Z1: Eine Begrenzung einer Höchstgeschwindigkeit des Fahrzeuges beziehungsweise einer Drehzahl der Brennkraftmaschine und/oder Z2: eine Reduzierung des Moments der Brennkraftmaschine und/oder Z3: eine Verhinderung eines vorgebbaren n-ten Motorstarts (n = vorgebbare Anzahl der noch zulässigen Motorstarts), wobei n = 0 sein kann, so dass bereits ein nächster Motorstart verhindert wird, oder Z4: eine Verhinderung eines vorgebbaren n-ten Motorstarts (n = vorgebbare Anzahl der noch zulässigen Motorstarts) im Kombination mit dem Erreichen einer vorgegebenen Fahrtstrecke (x), wobei nach dem Erreichen der vorgegebenen Fahrtstrecke (x) nur n-vorgebbare Motorstarts (n = vorgebbare Anzahl der noch zulässigen Motorstarts) erlaubt ist/sind, wobei n = 0 sein kann, so dass nach dem Erreichen der vorgegebenen Fahrtstrecke (x) bereits ein nächster Motorstart verhindert wird.

Erfindungsgemäß wird eine Anordnung eines Kraftstoff-Sensors in einem Kraftstoffversorgungssystem einer Brennkraftmaschine zur Durchführung des Verfahren zur CO₂-Zertifizierung und CO₂-abhängigen Homologation von Fahrzeugen unter Berücksichtigung mindestens eines Gestaltungsmerkmales des Fahrzeuges vorgeschlagen, wobei erfindungsgemäß vorgesehen ist, dass als Gestaltungsmerkmal des Fahrzeuges zumindest die festgestellte Verwendung eines CO₂-reduzierten Kraftstoffs anhand eines ermittelten spezifische Zusammensetzungs-Musters berücksichtigt wird, wobei der Kraftstoff-Sensor in dem Kraftstoffversorgungssystem in einem Kraftstofftank oder in einer Kraftstoffzuführungsleitung zwischen Kraftstofftank und Brennkraftmaschine angeordnet ist, wobei der Kraftstoff-Sensor eingerichtet ist, die chemischen Bestandteilen des Kraftstoffs zu ermitteln, um spezifische Zusammensetzungs-Muster zu ermitteln, anhand derer eine Unterscheidung durch Gegenüberstellung mit dem mindestens einen Vergleichs-Zusammensetzungs-Muster vorgenommen werden kann.

Die Erfindung wird nachfolgend anhand der zugehörigen Zeichnungen erläutert. Es zeigen:

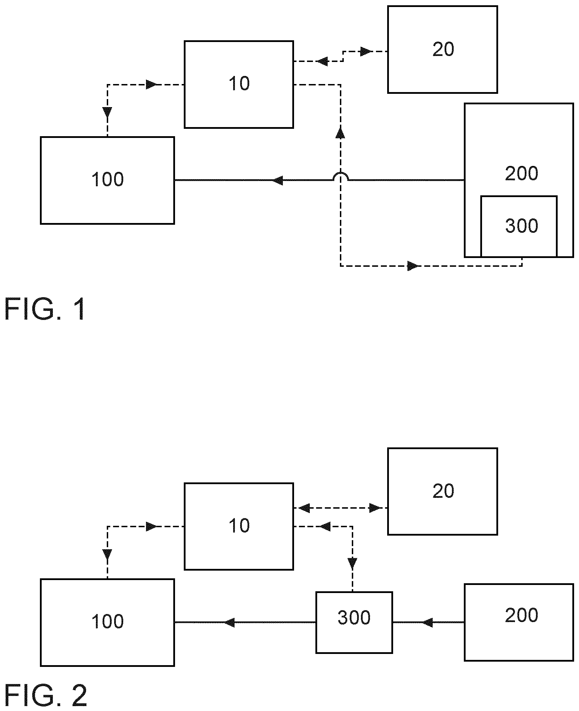

- Figur 1: eine schematische Darstellung der im Wesentlichen beteiligten Systemkomponenten in einer ersten Ausführungsform zur Verdeutlichung des Stoff- und Signalflusses eines Fahrzeuges, welches einem zugehörigen Kraftstoffversorgungssystem eine Sensierung der Kraftstoffzusammensetzung und/oder eines Markers ermöglicht,
- Figur 2: eine schematische Darstellung der im Wesentlichen beteiligten Systemkomponenten in einer ersten Ausführungsform zur Verdeutlichung des Stoff- und Signalflusses des Fahrzeuges, welches im zugehörigen Kraftstoffversorgungssystem eine Sensierung der Kraftstoffzusammensetzung und/oder eines Markers ermöglicht.

Die Erfindung wird in einer Zusammenschau der Figuren 1 und 2 erläutert, wobei in der Beschreibung entsprechend auf die Figuren Bezug genommen wird.

Die Erfindung geht von einem Kraftstoffversorgungssystem aus, dem eine entsprechende Steuereinrichtung, insbesondere ein Motor-Steuergerät 10, zugeordnet ist und das mit einer Brennkraftmaschine 100 und erfindungsgemäß mit einem speziellen Kraftstoff-Sensor 300 steuerungsseitig in Verbindung steht.

Das Kraftstoffversorgungssystem umfasst einen Kraftstofftank 200 und die üblichen weiteren, ebenfalls über das Motor-Steuergerät 10 ansteuerbaren Komponenten, die in den vereinfachten schematischen Darstellungen gemäß Figur 1 und 2 nicht dargestellt sind.

In Figur 1 ist der Kraftstoff-Sensor 300 im Kraftstofftank 200 angeordnet.

In Figur 2 ist der Kraftstoff-Sensor 300 in der Kraftstoffzuführungsleitung zwischen Kraftstofftank 200 und Brennkraftmaschine 100 angeordnet.

Prinzipiell besteht die Grundidee darin, dass der Kraftstoff-Sensor 300 mit dem Motor-Steuergerät 10 steuerungsseitig in Verbindung steht, wobei der Kraftstoff-Sensor 300 derart ausgebildet ist, dass er erkennt, welcher Kraftstoff sich im Kraftstofftank 200 befindet und beim Gebrauch der Brennkraftmaschine 100 aktuell verwendet wird.

Vorgesehen ist, dass über die Motorsteuerung die Signale des Kraftstoff-Sensors 300 dahingehend geprüft werden, ob sich ein CO₂-reduzierter Kraftstoff im Kraftstofftank 200 befindet, für den das Fahrzeug auch homologisiert (zugelassen) wurde.

Erfindungsgemäß ist vorgesehen, dass in dem Fall, dass mittels des Kraftstoff-Sensors 300 festgestellt wird, dass sich kein CO₂-reduzierter Kraftstoff im Kraftstofftank 200 befindet, über die Motorsteuerung Maßnahmen durchgeführt werden, die nachfolgend erläutert werden.
a) Der Benutzer wird über deutliche Meldungen in einem Anzeigeelement 20 des Kraftstoffversorgungssystems des Fahrzeuges über die Fehlbetankung informiert und wird aufgefordert, innerhalb einer weiteren vorgebbaren Fahrstrecke x (x = beispielsweise 100 km) die nächste Werkstatt aufzusuchen. In der Werkstatt wird das Kraftstoffversorgungssystem gereingt und das Fahrzeug wieder mit dem zugelassenen CO₂-reduzierten Kraftstoff befüllt, so dass die Betriebsbereitschaft wiederhergestellt ist.
   Bringt der Benutzer das Fahrzeug innerhalb der vorgegebenen Fahrstrecke (beispielsweise 100 km) nicht in die Werkstatt, erfolgen weitere Maßnahmen.
b) Der Benutzer wird über deutliche Meldungen in einem Anzeigeelement 20 des Kraftstoffversorgungssystems des Fahrzeuges über die Fehlbetankung und über durch die Fehlbetankung einschränkende Maßnahmen informiert, um den Benutzer dazu zu zwingen, das Fahrzeug in die Werkstatt zu bringen, damit das Kraftstoffversorgungssystem gereinigt und das Fahrzeug wieder mit dem zugelassenen Kraftstoff befüllt werden kann.

Folgende Zwangsmaßnahmen Zn werden vorgeschlagen:
Gemäß einer ersten Maßnahme Z1 erfolgt einer Begrenzung der Höchstgeschwindigkeit.

Gemäß einer zweiten Maßnahme Z2 wird eine Reduzierung des Moments der Brennkraftmaschine 100 und damit schlechte Fahrbarkeit des Fahrzeuges bewirkt.

Gemäß einer dritten Maßnahme Z3 werden nur noch n Motorstarts (n = vorgebbare Anzahl der noch zulässigen Motorstarts) erlaubt, wobei n = 0 sein kann, so dass bereits ein nächster Motorstart verhindert wird.

Gemäß einer vierten Maßnahme Z4 Kombination der dritten Maßnahme Z3 mit einer vorgebbaren noch x = km zulässigen Fahrtstrecke, wobei nach Erreichen der vorgegebenen Fahrtstrecke nur noch n Motorstarts (n = vorgebbare Anzahl der noch zulässigen Motorstarts) erlaubt ist/sind, wobei n = 0 sein kann, so dass bereits ein nächster Motorstart verhindert wird.

Der Kraftstoff-Sensor 300 und das zugehörige Analyseverfahren ist derart ausgebildet, so dass CO₂-reduzierte Kraftstoffe, insbesondere Bio- und Synthetikanteile von CO₂-reduzierten Kraftstoffen oder CO₂-reduzierende Zusätze oder CO₂-reduzierte Kraftstoffe anhand von spezifsichen Markern in dem spezifischen Zusammensetzungs-Muster durch den Vergleich mit dem mindestens einen als CO₂-reduzierten Kraftstoff standardisierten oder normierten Vergleichs-Zusammensetzungs-Muster möglich ist.
CO₂-reduzierte Kraftstoffe:
   - Biodiesel (beispielsweise R33 BlueDiesel der Anmelderin)
   - paraffinischer Diesel
   - Sweden Class
CO₂-reduzierende Zusätze:
   - OME (Polyoxymethylendimethylether)
   - Ethanol-Zusätze jeder Art
CO₂-reduzierte Kraftstoffe und/oder CO₂-reduzierende Zusätze signalisierende Marker
CO₂-reduzierter Kraftstoff mit chemischem oder anderweitig markierendem Marker

Vorgeschlagen wird, ein Homologationsverfahren einzusetzen, bei dem die CO₂-Werte eines Fahrzeuges, insbesondere eines PKW oder eines LKW, bestimmt werden.

Ein solches Verfahren besteht im Wesentlichen aus einem Berechnungstool, das auf dem Verrechnen von Komponenten, technischen Einrichtungen oder Gestaltungsmerkmalen mit einer Relevanz für die CO₂-Emmisionen des jeweiligen Fahrzeuges (PKW, LKW und so weiter) beruht.

Dieses Berechnungstool berücksichtigt bei dem Fahrzeug CO₂-reduzierende Gestaltungsmerkmale, insbesondere Merkmale der Aerodynamik, des Leichtbaus, des Wirkungsgrads des Motors und und/oder die Verwendung eines ökologisch erzeugten Ökostromanteiles einer Stromladung eines Plug-in-Hybrid-Fahrzeuges und auch gemäß einer Norm die Verwendung eines Kraftstoff, der als CO₂-reduzierter Kraftstoff eingeordnet wird, wobei gemäß dem erfindungsgemäßen Verfahren im Wesentlichen sichergestellt ist, dass ausschließlich der CO₂-reduzierte Kraftstoff und/oder ein ökologisch erzeugter Ökostromanteil einer Stromladung verwendet wird.

Es wird insbesondere vorgeschlagen, das sich derzeit in der Entwicklung befindende Berechnungstool der EU-Kommision VECTO (Vehicle Energy Consumption Calculation Tool) um das Merkmal der Verwendung eines CO₂-reduzierten Kraftstoffs in dem Fahrzeug zu erweitern und eine spezielle Homologation bestimmter "CO₂-reduzierter Fahrzeuge" einzuführen. Dadurch kann unter Berücksichtigung des erfindungsgemäßen Verfahrens und des zugehörigen Systems ein wirksamer Beitrag zur Verringerung der CO₂-Emissionen geleistet werden.

### Bezugszeichenliste

- 100: Brennkraftmaschine
- 200: Kraftstofftank
- 300: Kraftstoff-Sensor
- 10: Motor-Steuergerät
- 20: Anzeigeelement

## Patentansprüche

1. Verfahren zur CO₂-Zertifizierung und CO₂-abhängigen Homologation eines Fahrzeuges unter Berücksichtigung mindestens eines Gestaltungsmerkmales des Fahrzeuges, **wobei** als Gestaltungsmerkmal des Fahrzeuges die festgestellte Verwendung eines CO₂-reduzierten Kraftstoffs berücksichtigt wird, wobei über eine Motorsteuerung Signale eines Kraftstoff-Sensors (300) dahingehend geprüft werden, ob sich der CO₂-reduzierte Kraftstoff im Kraftstofftank (200) befindet, für den das Fahrzeug auch homologiert wurde,
• wobei eine festgestellte Verwendung des CO₂-reduzierten Kraftstoffs anhand eines ermittelten spezifischen Zusammensetzungs-Musters berücksichtigt wird, wobei ein Kraftstoff-Sensor (300) in einem Kraftstofftank (200) oder in einer Kraftstoffzuführungsleitung zwischen dem Kraftstofftank und einer Brennkraftmaschine in einem Kraftstoffversorgungssystem angeordnet ist,
• wobei der Kraftstoff-Sensor (300) eingerichtet ist, chemische Bestandteile des Kraftstoffs zu ermitteln, um das spezifische Zusammensetzungs-Muster zu ermitteln, anhand dessen eine Unterscheidung durch Gegenüberstellung mit mindestens einem Vergleichs-Zusammensetzungs-Muster vorgenommen wird,
• wobei, wenn das spezifische Zusammensetzungs-Muster mit dem Vergleichs-Zusammensetzungs-Muster übereinstimmt, durch Vergleich der Muster nicht-CO₂-reduzierter Kraftstoff von CO₂-reduziertem Kraftstoff unterschieden wird,
• wobei die ausschließliche Verwendung des CO₂-reduzierten Kraftstoffs in dem Fahrzeug weitestgehend sichergestellt wird, indem eine Benutzung des Fahrzeuges nur unter Verwendung des CO₂-reduzierten Kraftstoffs erlaubt wird, wobei
• in dem Fall, dass mittels des Kraftstoff-Sensors (300) festgestellt wird, dass sich kein CO₂-reduzierter Kraftstoff im Kraftstofftank (200) befindet, über die Motorsteuerung Maßnahmen durchgeführt werden, wobei über mindestens ein Anzeigeelement (20) ein Hinweis zur aktuellen Nichtverwendung des CO₂-reduzierten Kraftstoffs gestuft ausgegeben wird, wobei mindestens ein Hinweis (a), b)) zumindest eine Information über die erfolgte Fehlbetankung und eine Aufforderung zu einer korrigierenden Handlung umfasst, wobei
• a) in dem mindestens einen Anzeigeelement (20) des Kraftstoffversorgungssystems des Fahrzeuges in einer ersten Stufe über eine durch den Kraftstoff-Sensor (300) festgestellte Fehlbetankung informiert wird, wobei dazu aufgefordert wird, innerhalb einer vorgebbaren Fahrstrecke (x) die nächste Werkstatt aufzusuchen, oder
• b) in dem mindestens einen Anzeigeelement (20) des Kraftstoffversorgungssystems des Fahrzeuges in einer zweiten Stufe über die durch den Kraftstoff-Sensor (300) festgestellte Fehlbetankung informiert wird, wobei dazu aufgefordert wird, innerhalb der vorgebbaren Fahrstrecke (x) die nächste Werkstatt aufzusuchen, wobei ferner in dem Anzeigeelement (20) des Kraftstoffversorgungssystems des Fahrzeuges über durch die Fehlbetankung zukünftig eintretende einschränkende Maßnahmen (Zn) informiert wird, wobei
• hinsichtlich der Vermeidung der Umgehbarkeit der gestuften Hinweise (a), b)) wirksamkeitsgestufte Maßnahmen (Zn) eingeleitet werden, wie
• - Z1: eine Begrenzung einer Höchstgeschwindigkeit des Fahrzeuges beziehungsweise einer Drehzahl der Brennkraftmaschine (100) und/oder
• - Z2: eine Reduzierung des Moments der Brennkraftmaschine (100) und/oder
• - Z3: eine Verhinderung eines vorgebbaren n-ten Motorstarts, wobei n = 0 sein kann, so dass bereits ein nächster Motorstart verhindert wird, oder
• - Z4: eine Verhinderung des vorgebbaren n-ten Motorstarts in Kombination mit dem Erreichen der vorgegebenen Fahrtstrecke (x), wobei nach dem Erreichen der vorgegebenen Fahrtstrecke (x) nur n-vorgebbare Motorstarts erlaubt ist/sind, wobei n = 0 sein kann, so dass nach dem Erreichen der vorgegebenen Fahrtstrecke (x) bereits ein nächster Motorstart verhindert wird.

2. Verfahren nach Anspruch 1, **dadurch gekennzeichnet, dass** weitere CO₂-reduzierende Gestaltungsmerkmale der Aerodynamik und/oder des Leichtbaus und/oder des Wirkungsgrads des Motors und/oder die Verwendung eines ökologisch erzeugten Ökostromanteiles eines Plug-in-Hybrid-Fahrzeuges des zu zertifizierenden und zu homologiesierenden Fahrzeuges festgestellt und bei der CO₂-Zertifizierung und CO₂-abhängigen Homologation berücksichtigt werden.

3. Verfahren nach Anspruch 1, **dadurch gekennzeichnet, dass** mittels des Kraftstoff-Sensors (300) objektiv die chemische spezifische Zusammensetzung des Kraftstoffs festgestellt wird, wobei aus der chemischen spezifischen Zusammensetzung des Kraftstoffs ein spezifisches chemisches Zusammensetzungs-Muster erstellt wird, das mit dem mindestens einem von mehreren Vergleichs-Zusammensetzungs-Mustern CO₂-reduzierter Kraftstoffe verglichen wird, so dass bei einer Übereinstimmung auf den als CO₂-reduziert geltenden Kraftstoff rückgeschlossen wird.

4. Verfahren nach Anspruch 3, **dadurch gekennzeichnet, dass** die Unterscheidung des nicht-CO₂-reduzierten Kraftstoffs von dem CO₂-reduzierten Kraftstoff anhand mindestens eines im spezifischen chemischen Zusammensetzungs-Muster festgestellten Markers vorgenommen wird, dem als CO₂-reduzierten Kraftstoff zugelassenem Kraftstoff hinzugefügt wird, wobei der Marker auch im Vergleichs-Zusammensetzungs-Muster enthalten ist, so dass bei einer Übereinstimmung auf den als CO₂-reduziert geltenden Kraftstoff rückgeschlossen wird.

5. Verfahren nach Anspruch 1 und 4, **dadurch gekennzeichnet, dass** die chemischen Bestandteile des Kraftstoffs oder des mindestens einen Markers durch ein Analyseverfahren festgestellt werden, welches dem mindestens einen Kraftstoff-Sensor (300) zugeordnet ist, der die chemischen Bestandteile und/oder den mindestens einen hinzugefügten Marker auf chemische oder andere Weise erkennt.

6. Verfahren nach Anspruch 5, **dadurch gekennzeichnet, dass** ein vorgebbarer Schwellenwert derjenigen Anteile der chemischen Bestandteile des Kraftstoffs festgelegt wird, wobei der Schwellenwert überschritten werden muss, damit der Kraftstoff als CO₂-reduzierter Kraftstoff erkannt und eingestuft wird.

7. Verfahren nach Anspruch 1 und 5, **dadurch gekennzeichnet, dass** als Analyseverfahren zur Analyse der chemischen Bestandteile des Kraftstoffs und/oder des mindestens eines Markers ein Spektroskopieverfahren,
• eine NIR-Spektroskopie (NIR =Nahinfrarot-Spektroskopie) oder
• eine NMR-Spektroskopie (NMR = Nuklear Magnet-Resonanz-Spektroskopie) oder
• eine LIF-Spektroskopie (LIF = Laserinduzierte Fluoreszenz-Spektroskopie) oder
• eine ZLIF-Spektroskopie (ZLIF = zeitliche laserinduzierte Fuoreszenz-Spektroskopie)
durchgeführt wird, dem ein Kraftstoff-Sensor (300) zugeordnet ist, der die chemischen Bestandteile des Kraftstoffs und des mindestens einen Markers feststellt.

8. Verfahren nach Anspruch 4, **dadurch gekennzeichnet, dass** als Analyseverfahren zur Analyse des mindestens eines Markers ein dem jeweiligen Marker spezifisch zugeordnetes Analyseverfahren durchgeführt wird, dem ein spezifischer Kraftstoff-Sensor (300) zugeordnet ist, der ausschließlich den mindestens einen Marker feststellt.

9. Anordnung eines Kraftstoff-Sensors (300) in einem Kraftstoffversorgungssystem einer Brennkraftmaschine (100) zur Durchführung des Verfahrens nach mindestens einem der Ansprüche 1 bis 8 zur CO₂-Zertifizierung und CO₂-abhängigen Homologation von Fahrzeugen unter Berücksichtigung mindestens eines Gestaltungsmerkmales des Fahrzeuges, **dadurch gekennzeichnet, dass** als Gestaltungsmerkmal des Fahrzeuges zumindest die festgestellte Verwendung eines CO₂-reduzierten Kraftstoffs berücksichtigt wird,
wobei der Kraftstoff-Sensor (300)
• in einem Kraftstofftank (200) des Kraftstoffversorgungssystems oder
• in einer Kraftstoffzuführungsleitung des Kraftstoffversorgungssystems zwischen dem Kraftstofftank (200) und einer Brennkraftmaschine (100) angeordnet ist,
wobei der Kraftstoff-Sensor (300) eingerichtet ist, die chemischen Bestandteile des Kraftstoffs zu ermitteln, um spezifische Zusammensetzungs-Muster zu ermitteln, anhand denen eine Unterscheidung durch Gegenüberstellung mit mindestens einem Vergleichs-Zusammensetzungs-Muster vorgenommen wird.

## Claims

1. Method for CO₂ certification and CO₂-dependent homologation of a vehicle taking into account at least one design feature of the vehicle, **wherein** as a design feature of the vehicle the detected use of a CO₂-reduced fuel is taken into account, wherein signals from a fuel sensor (300) are checked via an engine control unit to determine whether there is CO₂-reduced fuel in the fuel tank (200), for which the vehicle has also been homologated,
• wherein a detected use of CO₂-reduced fuel is taken into account on the basis of a determined specific composition pattern, wherein a fuel sensor (300) is arranged in a fuel tank (200) or in a fuel supply line between the fuel tank and an internal combustion engine in a fuel supply system,
• wherein the fuel sensor (300) is configured to determine chemical components of the fuel in order to determine the specific composition pattern on the basis of which a distinction is made by comparison with at least one reference composition pattern,
• wherein if the specific composition pattern matches the reference composition pattern, by comparing the patterns non-CO₂-reduced fuel is distinguished from CO₂-reduced fuel,
• wherein the exclusive use of CO₂-reduced fuel in the vehicle is ensured as far as possible by allowing vehicle use only when using the CO₂-reduced fuel, wherein
• in the event that the fuel sensor (300) detects that there is no CO₂-reduced fuel in the fuel tank (200), measures are taken via the engine control unit, wherein at least one display element (20) indicates in stages the current non-use of CO₂-reduced fuel, wherein at least one indication (a), b)) comprises at least one piece of information about the incorrect refueling and a request for corrective action, wherein
• a) in the at least one display element (20) of the fuel supply system of the vehicle, information is provided in a first stage about an incorrect refueling detected by the fuel sensor (300), wherein a request is issued to visit the nearest workshop within a predeterminable driving distance (x), or
• b) in the at least one display element (20) of the fuel supply system of the vehicle, information is provided in a second stage about the incorrect refueling detected by the fuel sensor (300), wherein a request is issued to visit the nearest workshop within the predeterminable driving distance (x), wherein further information is provided in the display element (20) of the fuel supply system of the vehicle about future restrictive measures (Zn) that will occur as a result of the incorrect refueling, wherein
• with regard to avoiding the circumvention of the graded indications (a), b)), measures (Zn) graded in terms of effectiveness are introduced, such as
• - Z1: a limitation of a maximum speed of the vehicle or a rotational speed of the internal combustion engine (100) and/or
• - Z2: a reduction in the torque of the internal combustion engine (100) and/or
• - Z3: a prevention of a predeterminable nth engine start, where n = 0 is possible, so that a next engine start is already prevented, or
• - Z4: a prevention of the predeterminable nth engine start in combination with reaching the predefined driving distance (x), wherein after reaching the predefined driving distance (x) only n-predeterminable engine starts are permitted, where n = 0 is possible, so that after reaching the predefined driving distance (x) a next engine start is already prevented.

2. Method according to claim 1, **characterized in that** further CO₂-reducing design features of the aerodynamics and/or the lightweight construction and/or the efficiency of the engine, and/or the use of an ecologically generated green electricity share of a plug-in hybrid vehicle, of the vehicle to be certified and homologated are detected and are taken into account in the CO₂ certification and CO₂-dependent homologation.

3. Method according to claim 1, **characterized in that** the chemical specific composition of the fuel is objectively detected by means of the fuel sensor (300), a specific chemical composition pattern being created from the chemical specific composition of the fuel, which pattern is compared with the at least one of a plurality of reference composition patterns of CO₂-reduced fuels, so that if they match, the CO₂-reduced applicable fuel is concluded.

4. Method according to claim 3, **characterized in that** the non-CO₂-reduced fuel is distinguished from the CO₂-reduced fuel using at least one marker detected in the specific chemical composition pattern, which is added as CO₂-reduced fuel to the approved fuel, the marker also being included in the reference composition pattern, so that if they match, the CO₂-reduced applicable fuel is concluded.

5. Method according to claim 1 and 4, **characterized in that** the chemical components of the fuel or of the at least one marker are detected by an analytical method which is associated with the at least one fuel sensor (300) which identifies the chemical components and/or the at least one added marker chemically or otherwise.

6. Method according to claim 5, **characterized in that** a predeterminable threshold value is set for the proportions of the chemical components of the fuel, which threshold value must be exceeded for the fuel to be identified and classified as CO₂-reduced fuel.

7. Method according to claim 1 and 5, **characterized in that** as an analytical method for analyzing the chemical components of the fuel and/or the at least one marker, a spectroscopy method,
• NIR spectroscopy (NIR = near-infrared spectroscopy) or
• NMR spectroscopy (NMR = nuclear magnetic resonance spectroscopy) or
• LlF spectroscopy (LIF = laser-induced fluorescence spectroscopy) or
• TR-LIF spectroscopy (TR-LIF = time-resolved laser-induced fluorescence spectroscopy)
is carried out, to which a fuel sensor (300) is assigned, which detects the chemical components of the fuel and the at least one marker.

8. Method according to claim 4, **characterized in that** as an analytical method for analyzing the at least one marker, an analytical method specifically assigned to the relevant marker is carried out, to which a specific fuel sensor (300) is assigned, which exclusively detects the at least one marker.

9. Arrangement of a fuel sensor (300) in a fuel supply system of an internal combustion engine (100) for carrying out the method according to at least one of claims 1 to 8 for CO₂ certification and CO₂-dependent homologation of vehicles taking into account at least one design feature of the vehicle,
**characterized in that** as a design feature of the vehicle at least the detected use of a CO₂-reduced fuel is taken into account,
the fuel sensor (300)
• being arranged in a fuel tank (200) of the fuel supply system or
• in a fuel supply line of the fuel supply system between the fuel tank (200) and an internal combustion engine (100),
the fuel sensor (300) being configured to determine the chemical components of the fuel in order to determine specific composition patterns on the basis of which a distinction is made by comparison with at least one reference composition pattern.

## Revendications

1. Procédé de certification de CO₂ et d'homologation dépendant du CO₂ d'un véhicule en tenant compte d'au moins une caractéristique de conception du véhicule, **dans lequel** l'utilisation constatée d'un carburant à teneur réduite en CO₂ est prise en compte comme caractéristique de conception du véhicule, dans lequel des signaux d'un capteur de carburant (300) sont contrôlés par l'intermédiaire d'une commande de moteur pour savoir si le carburant à teneur réduite en CO₂ se trouve dans le réservoir de carburant (200), pour lequel le véhicule a également été homologué,
• dans lequel une utilisation constatée du carburant à teneur réduite en CO₂ est prise en compte à l'aide d'un modèle de composition spécifique déterminé, dans lequel un capteur de carburant (300) est agencé dans un réservoir de carburant (200) ou dans une conduite d'alimentation en carburant entre le réservoir de carburant et un moteur à combustion interne dans un système d'alimentation en carburant,
• dans lequel le capteur de carburant (300) est conçu pour déterminer les composants chimiques du carburant afin de déterminer le modèle de composition spécifique à l'aide duquel une distinction est effectuée par opposition avec au moins un modèle de composition de comparaison,
• dans lequel, si le modèle de composition spécifique correspond au modèle de composition de comparaison, on distingue le carburant à teneur non réduite en CO₂ du carburant à teneur réduite en CO₂ en comparant les modèles,
• dans lequel l'utilisation exclusive du carburant à teneur réduite en CO₂ dans le véhicule est assurée dans la mesure du possible en permettant une utilisation du véhicule uniquement en utilisant le carburant à teneur réduite en CO₂, dans lequel
• dans le cas où il est constaté au moyen du capteur de carburant (300) qu'il n'y a pas de carburant à teneur réduite en CO₂ dans le réservoir de carburant (200), des mesures sont exécutées par l'intermédiaire de la commande du moteur, dans lequel une indication concernant la non-utilisation actuelle du carburant à teneur réduite en CO₂ est émise de manière échelonnée par l'intermédiaire d'au moins un élément d'affichage (20), dans lequel au moins une indication (a), b)) comprend au moins une information sur l'erreur de remplissage effectuée et une invitation à une action corrective, dans lequel
• a) dans l'au moins un élément d'affichage (20) du système d'alimentation en carburant du véhicule, on est informé dans une première étape d'une erreur de remplissage constatée par le capteur de carburant (300), en étant invité à se rendre dans l'atelier le plus proche dans une distance parcourue (x) pouvant être prédéfinie, ou
• b) dans l'au moins un élément d'affichage (20) du système d'alimentation en carburant du véhicule, on est informé dans une deuxième étape de l'erreur de remplissage constatée par le capteur de carburant (300), en étant invité à se rendre dans l'atelier le plus proche à l'intérieur de la distance parcourue (x) pouvant être prédéfinie, en étant informé en outre dans l'élément d'affichage (20) du système d'alimentation en carburant du véhicule de mesures restrictives (Zn) qui se produiront à l'avenir en raison de l'erreur de remplissage, dans lequel
• en ce qui concerne la prévention de la possibilité de contournement des indications échelonnées (a), b)), des mesures à effet échelonné (Zn) sont engagées, telles que
• Z1 : une limitation d'une vitesse maximale du véhicule, respectivement d'une vitesse de rotation du moteur à combustion interne (100) et/ou
• Z2 : une réduction du couple du moteur à combustion interne (100) et/ou
• Z3 : un empêchement d'un n-ième démarrage du moteur pouvant être prédéfini, dans lequel n peut être égal à 0, de sorte qu'un prochain démarrage du moteur est déjà empêché, ou
• Z4 : un empêchement du n-ième démarrage de moteur pouvant être prédéfini en combinaison avec l'atteinte de la distance parcourue (x) prédéfinie, dans lequel, après l'atteinte de la distance parcourue (x) prédéfinie, seuls n démarrages de moteur pouvant être prédéfinis sont autorisés, dans lequel n peut être égal à 0, de sorte qu'après l'atteinte de la distance parcourue (x) prédéfinie, un prochain démarrage de moteur est déjà empêché.

2. Procédé selon la revendication 1, **caractérisé en ce que** d'autres caractéristiques de conception réduisant le CO₂ de l'aérodynamique et/ou de la construction légère et/ou du rendement du moteur et/ou l'utilisation d'une part d'électricité verte produite de manière écologique d'un véhicule hybride rechargeable du véhicule à certifier et à homologuer sont constatées et prises en compte lors de la certification de CO₂ et de l'homologation dépendant du CO₂.

3. Procédé selon la revendication 1, **caractérisé en ce que** la composition chimique spécifique du carburant est constatée objectivement au moyen du capteur de carburant (300), dans lequel un modèle de composition chimique spécifique est établi à partir de la composition chimique spécifique du carburant, lequel modèle est comparé à au moins l'un de plusieurs modèles de composition de comparaison de carburants à teneur réduite en CO₂, de sorte que, en cas de concordance, on en déduit le carburant considéré comme étant à teneur réduite en CO₂.

4. Procédé selon la revendication 3, **caractérisé en ce que** la distinction entre le carburant à teneur non réduite en CO₂ et le carburant à teneur réduite en CO₂ est effectuée à l'aide d'au moins un marqueur constaté dans le modèle de composition chimique spécifique ajouté au carburant autorisé comme carburant à teneur réduite en CO₂, dans lequel le marqueur est également contenu dans le modèle de composition de comparaison, de sorte que, en cas de concordance, on en déduit le carburant considéré comme étant à teneur réduite en CO₂.

5. Procédé selon les revendications 1 et 4, **caractérisé en ce que** les composants chimiques du carburant ou de l'au moins un marqueur sont détectés par un procédé d'analyse associé à l'au moins un capteur de carburant (300) qui détecte les composants chimiques et/ou l'au moins un marqueur ajouté par voie chimique ou autre.

6. Procédé selon la revendication 5, **caractérisé en ce qu'**une valeur seuil pouvant être prédéfinie des proportions des composants chimiques du carburant est constatée, dans lequel la valeur seuil doit être dépassée pour que le carburant soit reconnu et classé comme carburant à teneur réduite en CO₂.

7. Procédé selon les revendications 1 et 5, **caractérisé en ce que**, comme procédé d'analyse pour l'analyse des composants chimiques du carburant et/ou de l'au moins un marqueur, un procédé de spectroscopie,
• une spectroscopie NIR (NIR = proche infrarouge) ou
• une spectroscopie RMN (RMN = spectroscopie par résonance magnétique nucléaire) ou
• une spectroscopie LIF (LIF = spectroscopie de fluorescence induite par laser) ou
• une spectroscopie ZLIF (ZLIF = spectroscopie de fluorescence induite par laser dans le temps)
est effectué(e), à laquelle est associé un capteur de carburant (300) qui constate les composants chimiques du carburant et de l'au moins un marqueur.

8. Procédé selon la revendication 4, **caractérisé en ce que**, comme procédé d'analyse pour analyser l'au moins un marqueur, un procédé d'analyse spécifiquement associé au marqueur respectif est effectué, auquel est associé un capteur de carburant (300) spécifique qui constate exclusivement l'au moins un marqueur.

9. Agencement d'un capteur de carburant (300) dans un système d'alimentation en carburant d'un moteur à combustion interne (100) pour la réalisation du procédé selon au moins l'une des revendications 1 à 8 de certification de CO₂ et d'homologation dépendant du CO₂ de véhicules en tenant compte d'au moins une caractéristique de conception du véhicule, **caractérisé en ce qu'**au moins l'utilisation constatée d'un carburant à teneur réduite en CO₂ est prise en compte comme caractéristique de conception du véhicule,
dans lequel le capteur de carburant (300) est agencé
• dans un réservoir de carburant (200) du système d'alimentation en carburant, ou
• dans une conduite d'alimentation en carburant du système d'alimentation en carburant entre le réservoir de carburant (200) et un moteur à combustion interne (100),
dans lequel le capteur de carburant (300) est conçu pour déterminer les composants chimiques du carburant afin de déterminer des modèles de composition spécifiques à l'aide desquels une distinction est effectuée par opposition avec au moins un modèle de composition de comparaison.
